# EUROPEAN PATENT SPECIFICATION

(11) **EP 3 701 934 B1**
(45) Date of publication and mention of the grant of the patent: **17.01.2024**
(21) Application number: 18860645.3
(22) Date of filing: 20.09.2018
(51) Int. Cl.: A61K 8/49, A61Q 19/08, C07K 14/575, A61K 8/67

(54) **COMPOSITION FOR PROMOTING ADIPOCYTE DIFFERENTIATION OR ADIPONECTIN, COMPRISING TRIMETHOXY PHENYL COMPOUND**
ZUSAMMENSETZUNG ZUR FÖRDERUNG DER ADIPOZYTENDIFFERENZIERUNG ODER ADIPONECTIN MIT EINER TRIMETHOXYPHENYLVERBINDUNG
COMPOSITION POUR FAVORISER LA DIFFÉRENCIATION ADIPOCYTAIRE OU LA PRODUCTION D'ADIPONECTINE, COMPRENANT UN COMPOSÉ DE TRIMÉTHOXY PHÉNYLE

(30) Priority: 29.09.2017 KR 20170127407
(43) Date of publication of application: 02.09.2020
(73) Proprietor: Amorepacific Corporation, Seoul 04386 (KR)
(72) Inventor: PARK, Pil Joon, Yongin-si Gyeonggi-do 17074 (KR); LEE, Tae Ryong, Yongin-si Gyeonggi-do 17074 (KR); CHO, Eungyung, Yongin-si Gyeonggi-do 17074 (KR)
(74) Representative: Germain Maureau
(86) International application number: PCT/KR2018/011124
(87) International publication number: WO 2019/066380

(56) References cited:
- EP-A1- 0 419 901
- EP-A1- 3 097 913
- WO-A1-2015/108394
- WO-A1-2017/095098
- WO-A1-2019/059555
- JP-A- 2009 051 796
- KR-A- 20160 020 212
- KR-B1- 960 016 127
- COSTA, F. L. P. et al.: "Conformational Analysis, Experimental and GIAO-DFT 13C NMR Chemical Shift Calculation on 2'-hydroxy-3,4,5-trimethoxy-chalcone", Journal of the Brazilian Chemical Society, vol. 28, no. 11, 31 March 2017 (2017-03-31), pages 2130-2135, XP055586110, ISSN: 0103-5053, DOI: 10.21577/0103-5053.20170060
- RAMKUMAR, K. et al.: "Discovery of 3-acetyl-4-hydroxy-2-pyranone Derivatives and Their Difluoridoborate Complexes as a Novel Class of HIV-1 Integrase Inhibitors", Bioorganic & Medicinal Chemistry, vol. 16, 30 August 2008 (2008-08-30), pages 8988-8998, XP025468958, DOI: doi:10.1016/j.bmc.2008.08.067

## Description

### [Technical Field]

Disclosed is a composition for promoting adipocyte differentiation or adiponectin production, which contains a trimethoxyphenyl compound as defined in claim 1, a salt thereof, a hydrate thereof or a solvate thereof.

### [Background Art]

The basic structure of skin is maintained by subcutaneous adipose tissue. The subcutaneous adipose tissue plays a critical role in maintaining the volume and strength of the skin. Accordingly, increasing the volume of adipose tissue can be a fundamental solution for maintaining the volume and elasticity of skin, rather than the existing methods of providing elasticity to the outer dermal or epidermal layer of the skin.

Specifically, with aging, wrinkles are formed on human skin and skin elasticity is increased at the same time. Skin aging such as wrinkle formation and decreased elasticity is a complicated phenomenon caused by the degradation and decrease of skin fibers such as collagen and elastin, which constitute the skin, as well as the decreased activity of adipocytes and the decrease in lipid droplets resulting therefrom. Therefore, skin wrinkles and elasticity may be improved if the lipid droplets can be produced and accumulated by promoting the differentiation of human adipocytes.

Meanwhile, adiponectin is a protein hormone specifically secreted by adipocytes. It is one of adipokines and plays an important role in regulating cardiovascular diseases such as hyperglycemia, hyperinsulinemia, obesity and arteriosclerosis by enhancing the function of insulin and inducing insulin resistance. In addition, adiponectin has a role in suppressing metastasis of cancer cells and inflammatory responses.

Especially, adiponectin heals wound, inhibits fibrosis, improves skin wrinkles, moisturizes skin, etc. by promoting not only the proliferation of keratinocytes but also the expression of filaggrin, hyaluronic acid and extracellular matrix in the skin.

EP 3 097 913 A1 discloses a cosmetic composition containing a hydroxypyranone derivative compound for promoting differentiation of adipocytes in a skin layer, which thus increases the skin volume or elasticity.

WO 2017/095098 A1 discloses a novel hydroxyl pyranone compound for increasing skin volume or elasticity.

However, since the existing substances for promoting adipocyte differentiation or adiponectin production do not exhibit sufficient effect of enhancing the volume and elasticity of skin human body when contained in cosmetic compositions, etc., they cannot fully satisfy the users of the cosmetic compositions, etc.

Accordingly, through the experimental results on human-derived subcutaneous fat, it is necessary to develop a composition for promoting adipocyte differentiation or adiponectin production with improved effect.

### [Disclosure]

### [Technical Problem]

The promotion of adipocyte differentiation or adiponectin production is a very important factor in maintaining the volume and elasticity of skin. The inventors of the present disclosure have experimentally identified that a composition containing a novel trimethoxyphenyl compound as defined in claim 1 promotes adipocyte differentiation or adiponectin production, and have completed the present disclosure.

Therefore, the present disclosure is directed to providing a composition for promoting adipocyte differentiation or adiponectin production, which contains a trimethoxyphenyl compound, a pharmaceutically acceptable salt thereof, a hydrate thereof or a solvate thereof as an active ingredient.

### [Technical Solution]

In accordance with the present invention, it is provided a non-therapeutic use of a composition for enhancing volume or elasticity of skin by promoting adipocyte differentiation or adiponectin production, which contains a trimethoxyphenyl compound as defined in claim 1, a pharmaceutically acceptable salt thereof, a hydrate thereof or a solvate thereof as an active ingredient. Compositions according to the present disclosure are not part of the invention as claimed.

### [Advantageous Effects]

A composition for promoting adipocyte differentiation or adiponectin production, which contains a trimethoxyphenyl compound as defined in claim 1, a pharmaceutically acceptable salt thereof, a hydrate thereof or a solvate thereof as an active ingredient, of the present disclosure promotes adipocyte differentiation or adiponectin production. Accordingly, the composition can be variously used as a pharmaceutical composition, a cosmetic composition or a formulation for external application to skin for promoting adipocyte differentiation or adiponectin production and enhancing skin volume or elasticity.

### [Brief Description of Drawings]

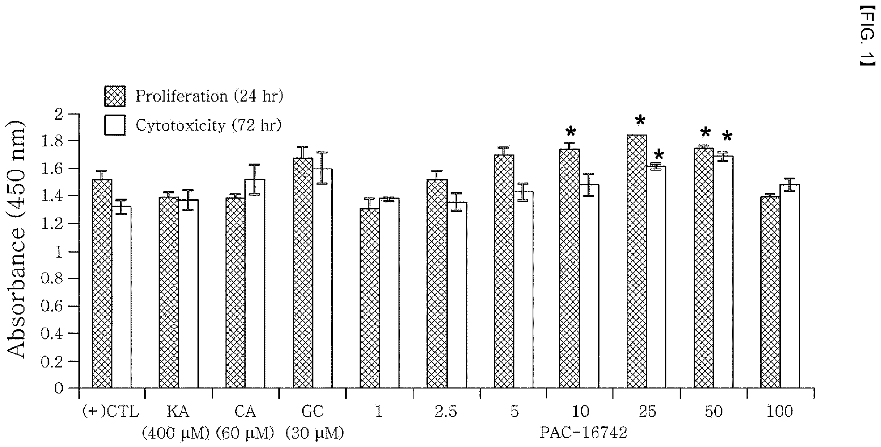

FIG. 1 shows a result of treating human-derived subcutaneous adipocytes with a composition containing a trimethoxyphenyl compound (hereinafter, PAC-16742) according to an exemplary embodiment of the present disclosure or with kojic acid, cinnamic acid and glibenclamide as positive control groups and analyzing cell proliferation and cytotoxicity.

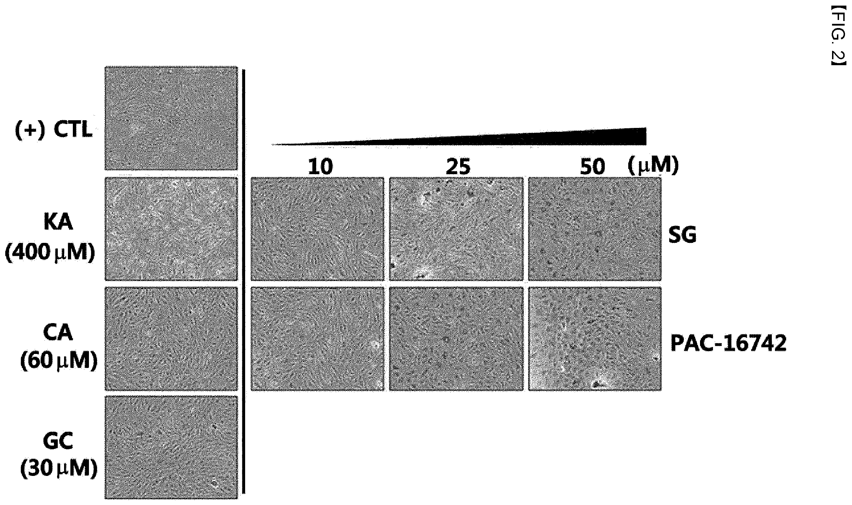

FIG. 2 shows a result of treating human-derived subcutaneous adipocytes with a composition containing a trimethoxyphenyl compound (PAC-16742) according to an exemplary embodiment of the present disclosure or with kojic acid, cinnamic acid and glibenclamide as positive control groups and investigating the quantity of proliferated adipocytes by staining with Oil Red O (ORO).

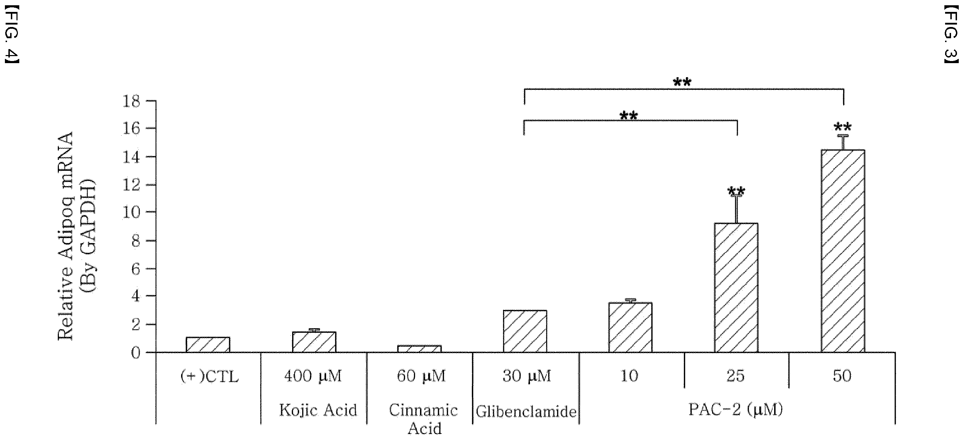

FIG. 3 shows a result of treating human subcutaneous adipocytes with a composition containing a trimethoxyphenyl compound (PAC-2) according to an exemplary embodiment of the present disclosure or with kojic acid, cinnamic acid and glibenclamide as positive control groups and measuring the mRNA expression level of adiponectin (Hereinafter, Adipoq).

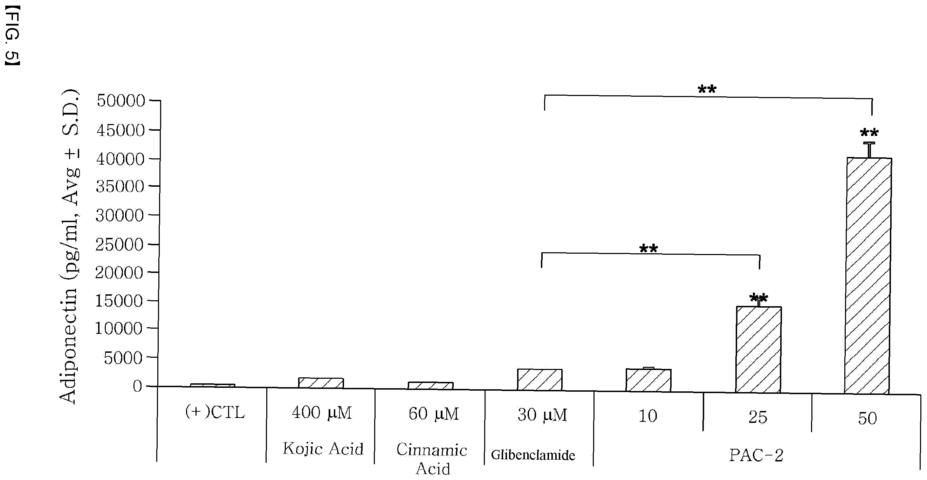

FIG. 4 shows a result of treating human subcutaneous adipocytes with a composition containing a trimethoxyphenyl compound (PAC-2) according to an exemplary embodiment of the present disclosure or with kojic acid, cinnamic acid and glibenclamide as positive control groups and measuring the quantity of adiponectin production by enzyme-linked immunosorbent assay (ELISA assay).

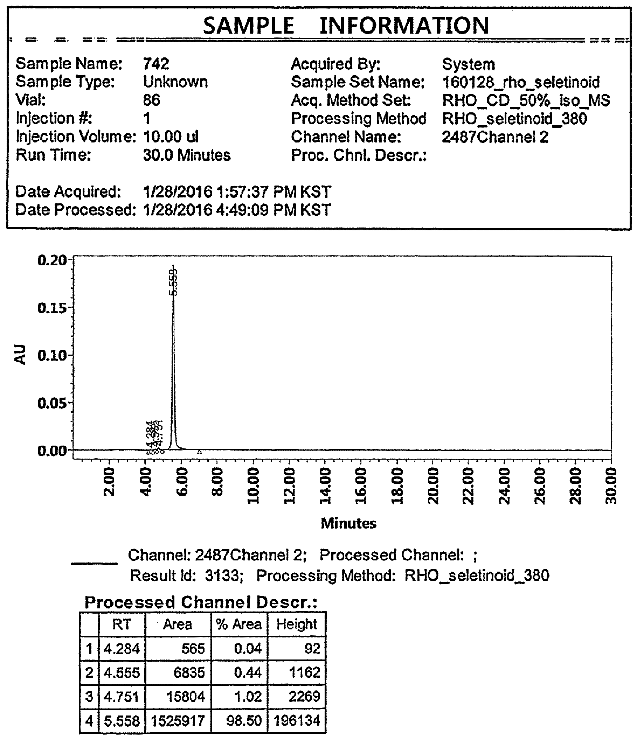

FIG. 5 shows an NMR analysis result of a trimethoxyphenyl compound (PAC-16742, PAC-2) according to an exemplary embodiment of the present disclosure (sample name: 742).

### [Best Mode]

### Definition of terms

In the present disclosure, when a certain portion is described to "include" a certain element, it does not preclude the existence of another element unless explicitly stated otherwise.

### Description of exemplary embodiments

Hereinafter, the present disclosure is described in detail.

The present disclosure provides a composition for promoting adipocyte differentiation or adiponectin production, which contains a trimethoxyphenyl compound represented by Chemical Formula 1, a pharmaceutically acceptable salt thereof, a hydrate thereof or a solvate thereof as an active ingredient.

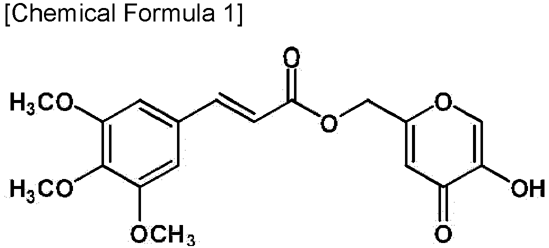

The IUPAC name of the trimethoxyphenyl compound of Chemical Formula 1 is 3-(3,4,5-trimethoxyphenyl)-acrylic acid 5-hydroxy-4-oxo-4H-pyran-2-ylmethyl ester.

In the present disclosure, "essentially pure" means, for example, when used in connection with enantiomers or diastereomers, that a specific compound as an example of the enantiomer or the diastereomer is present in about 90% (w/v) or more, specifically about 95% or more, more specifically about 97% or more or about 98% or more, further more specifically about 99% or more, even more specifically about 99.5% or more.

In the present disclosure, "pharmaceutically acceptable" means being approved or approvable by a government or a regulatory organization, which is equivalent thereto, or being listed in the Pharmacopoeia or other generally recognized pharmacopoeia for use in animals, more specifically in humans, since significant toxic effect can be avoided when used with a common medicinal dosage.

In the present disclosure, the "pharmaceutically acceptable salt" means a salt which is pharmaceutically acceptable and has the desired pharmacological activity of a parent compound. The salt may include: (1) an acid addition salt formed from an inorganic acid such as hydrochloric acid, hydrobromic acid, sulfuric acid, nitric acid, phosphoric acid, etc., or from an organic acid such as acetic acid, propionic acid, hexanoic acid, cyclopentylpropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid and muconic acid; or (2) a salt formed as the acidic proton present in the parent compound is replaced.

In the present disclosure, the "hydrate" means a compound to which water is bound. The term is used in a broad concept, including an inclusion compound with no chemical bonding between water and the compound.

In the present disclosure, the "solvate" means a higher-order compound formed between a solute molecule or ion and a solvent molecule or ion.

The compound of Chemical Formula 1 is a pale-yellow solid compound at room temperature.

The compound represented by Chemical Formula 1 is prepared by reacting a pyranone derivative compound of Chemical Formula 2 with a trimethoxyphenyl ester compound of Chemical Formula 3 as shown in Scheme 1: 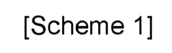 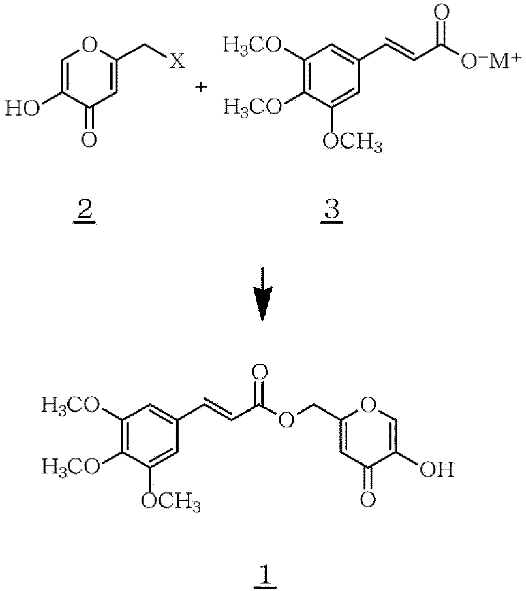 wherein X is a halogen element, and M is Li, Na or K.

Referring to Scheme 1, the compound of Chemical Formula 1 is prepared by bonding between the halogen element of the pyranone compound and the metal of the trimethoxyphenyl ester compound.

In the pyranone compound of Chemical Formula 2, X is a halogen element. The halogen element may be F, Cl, Br or I, specifically Cl. The compound of Chemical Formula 2 may be purchased commercially or prepared directly.

In an exemplary embodiment of the present disclosure, the compound may be 5-hydroxy-2-(chloromethyl)-4H-pyran-4-one wherein X is Cl, and it may be prepared by reacting 5-hydroxy-2-(hydroxymethyl)-4H-pyran-4-one with thionyl chloride (SOCl₂).

And, the trimethoxyphenyl ester compound of Chemical Formula 3 may be an ionization salt of 3-(3,4,5-trimethoxyphenyl)-acrylic acid, and may be present in the form wherein a cation (M⁺) is bound to the carboxyl group of 3-(3,4,5-trimethoxyphenyl)-acrylic acid.

The cation (M⁺) may be one selected from a group consisting of Li⁺, Na⁺ and K⁺. Specifically, the compound of Chemical Formula 3 may be an ionization product of 3-(3,4,5-trimethoxyphenyl)-acrylic acid and Na⁺, and it may be prepared, for example, by ionizing 3-(3,4,5-trimethoxyphenyl)-acrylic acid and sodium hydroxide by dissolving them together in methanol and then distilling the methanol.

In a specific exemplary embodiment of the present disclosure, the compound of Chemical Formula 2 may be 5-hydroxy-2-(chloromethyl)-4H-pyran-4-one and the compound of Chemical Formula 3 may be sodium 3-(3,4,5-trimethoxyphenyl)-acryl acid. In addition, the trimethoxyphenyl compound of Chemical Formula 1 may be prepared through esterification of these compounds.

The reaction may be conducted under a condition where the halogen-metal reaction can be achieved enough, although not being particularly limited thereto.

The reaction may be conducted at the reflux temperature of the solvent, e.g., at 50-250 °C, for 0.5-5 hours, specifically for 1-3 hours.

The solvent may be any one in which the compounds of Chemical Formulas 2 and 3 can be dissolved sufficiently. For example, one selected from a group consisting of N,N-dimethylformamide (DMF), tetrahydrofuran (THF), dimethyl sulfoxide (DMSO), acetonitrile, dioxane, benzene, toluene, ether, methanol, hexane, cyclohexane, pyridine, N-methylpyrrolidone and a combination thereof may be used. Specifically, DMF may be used.

After the reaction, a high-purity compound may be obtained by distilling the solvent and conducting a usual post-treatment process such as washing, drying, purification, etc.

The trimethoxyphenyl compound of Chemical Formula 1 according to the present disclosure can be used in various applications, specifically as an active ingredient of a cosmetic composition. Specifically, the trimethoxyphenyl compound may be usefully used as an active ingredient in a pharmaceutical composition, a cosmetic composition or a formulation for external application to skin for enhancing the volume or elasticity of skin by promoting adipocyte differentiation or adiponectin production.

Particularly, in the test examples described later, in experiments on human-derived subcutaneous adipocytes, rather than on animals such as rat, it was clearly demonstrated that when the composition is used as a cosmetic composition, a pharmaceutical composition, etc. for use in human, it provides an effect of promoting adipocyte differentiation or adiponectin production.

In an exemplary embodiment, the concentration of the active ingredient may be 1-100 µM, e.g., 10 µM or higher, 15 µM or higher, 20 µM or higher, 25 µM or higher, 30 µM or higher, 35 µM or higher or 40 µM or higher, and 90 µM or lower, 80 µM or lower, 70 µM or lower or 60 µM or lower, based on the total volume of the composition. Specifically, in an exemplary embodiment of the present disclosure, the concentration of the active ingredient may be 50 µM.

If the concentration is lower than 1 µM, the effect of promoting adipocyte differentiation may be insignificant. And, if it exceeds 100 µM, cytotoxicity may occur.

In an exemplary embodiment, the composition may be a pharmaceutical composition, a cosmetic composition or a formulation for external application to skin.

The pharmaceutical composition for promoting adipocyte differentiation or adiponectin production according to an exemplary embodiment of the present disclosure may further contain a pharmaceutical adjuvant such as an antiseptic, a stabilizer, a wetting agent, an emulsification accelerator, a salt and/or buffer for control of osmotic pressure, etc. and other therapeutically useful substances, and may be prepared into various formulations for oral administration or parenteral administration according to common methods.

The formulation for oral administration may be, for example, a tablet, a pill, a hard or soft capsule, a liquid, a suspension, an emulsion, a syrup, a powder, a fine granule, a granule, a pellet, etc., and these formulations may contain, in addition to the active ingredient, a surfactant, a diluent (e.g., lactose, dextrose, sucrose, mannitol, sorbitol, cellulose or glycine), a lubricant (e.g., silica, talc, stearic acid and its magnesium or calcium salt, or polyethylene glycol). A tablet may further contain a binder such as magnesium aluminum silicate, starch paste, gelatin, tragacanth, methylcellulose, sodium carboxymethylcellulose and polyvinylpyrrolidone, and may optionally contain pharmaceutical additives such as a disintegrant such as starch, agar, alginic acid or its sodium salt, an absorbent, a colorant, a flavor, a sweetener, etc. The tablet may be prepared by common mixing, granulation or coating methods. The formulation for parenteral administration may be a formulation for transdermal administration, and may be, for example, an injection, a medicinal drop, an ointment, a lotion, a gel, a cream, a spray, a suspension, an emulsion, a suppository, a patch, etc., although not being limited thereto.

The pharmaceutical composition according to an exemplary embodiment of the present disclosure may be administered parenterally, rectally, topically, transdermally, subcutaneously, etc.

The pharmaceutical composition may promote adipocyte differentiation or adiponectin production.

Determination of the administration dosage of the active ingredient is within the level of those of ordinary skill. The daily administration dosage varies depending on various factors such as the progress of a disease, the duration of the disease, the age and health condition of a subject, the presence of complications, etc. For an adult, the composition may be administered at a dosage of 1 µg/kg-100 mg/kg, e.g., 0.1-20 mg/kg, 0.5-20 mg/kg or 1-20 mg/kg, specifically 5-10 mg/kg, once to three times a day. However, the administration dosage does not limit the scope of the present disclosure by any means.

The composition for promoting adipocyte differentiation or adiponectin production according to an exemplary embodiment of the present disclosure may be a cosmetic composition. The cosmetic composition may contain a cosmetically or dermatologically acceptable medium or base. The composition may be provided in the form of any topically applicable formulation, e.g., a solution, a gel, a solid, an anhydrous paste, an oil-in-water emulsion, a suspension, a microemulsion, a microcapsule, a microgranule, an ionic (liposome) or non-ionic vesicular dispersion, a cream, a skin lotion, a powder, an ointment, a spray or a conceal stick. These compositions may be prepared according to common methods in the art. The composition according to the present disclosure may also be provided in the form of a foam or an aerosol composition further containing a compressed propellant.

The cosmetic composition according to an exemplary embodiment of the present disclosure is not particularly limited in its formulation. For example, it may be formulated into cosmetics, e.g., a softening lotion, an astringent lotion, a nourishing lotion, a nourishing cream, a massage cream, an essence, an eye cream, an eye essence, a cleansing cream, a cleansing foam, a cleansing water, a pack, a powder, a body lotion, a body cream, a body oil, a body essence, etc.

When the formulation of the present disclosure is a paste, a cream or a gel, an animal fiber, a plant fiber, a wax, paraffin, starch, tragacanth, a cellulose derivative, polyethylene glycol, silicone, bentonite, silica, talc, zinc oxide, etc. may be used as a carrier ingredient.

When the formulation of the present disclosure is a powder or a spray, lactose, talc, silica, aluminum hydroxide, calcium silicate or polyamide powder may be used as a carrier ingredient. In particular, when the formulation is a spray, it may further contain a propellant such as chlorofluorohydrocarbon, propane/butane or dimethyl ether.

When the formulation of the present disclosure is a solution or an emulsion, a solvent, a solubilizer or an emulsifier such as water, ethanol, isopropanol, ethyl carbonate, ethyl acetate, benzyl alcohol, benzyl benzoate, propylene glycol, 1,3-butylene glycol, an aliphatic glycerol ester, polyethylene glycol or a fatty acid ester of sorbitan may be used as a carrier ingredient.

When the formulation of the present disclosure is a suspension, a liquid diluent such as water, ethanol or propylene glycol, a suspending agent such as ethoxylated isostearyl alcohol, polyoxyethylene sorbitol ester, polyoxyethylene sorbitan ester, microcrystalline cellulose, aluminum metahydroxide, bentonite, agar, tragacanth, etc. may be used as a carrier ingredient.

When the formulation of the present disclosure is a surfactant-containing cleanser, an aliphatic alcohol sulfate, an aliphatic alcohol ether sulfate, sulfosuccinic acid monoester, isethionate, an imidazolinium derivative, methyl taurate, sarcosinate, a fatty acid amide ether sulfate, an alkylamido betaine, aliphatic alcohol, a fatty acid glyceride, a fatty acid diethanolamide, a vegetable oil, a lanolin derivative, an ethoxylated glycerol fatty acid ester, etc. may be used as a carrier ingredient.

The cosmetic composition according to an exemplary embodiment of the present disclosure may further contain functional additives and ingredients contained in general cosmetic compositions, in addition to the active ingredient. The functional additive may include an ingredient selected from a group consisting of a water-soluble vitamin, an oil-soluble vitamin, a polypeptide, a polysaccharide, a sphingolipid and a seaweed extract.

The cosmetic composition of the present disclosure may further contain, together with the functional additives, ingredients contained in general cosmetic compositions. The further contained ingredients may include an oil or fat, a humectant, an emollient, a surfactant, an organic or inorganic pigment, an organic powder, a UV absorbent, an antiseptic, a sterilizer, an antioxidant, a plant extract, a pH control agent, an alcohol, a colorant, a fragrance, a blood circulation promoter, a cooling agent, an antiperspirant, purified water, etc.

In addition, the composition for promoting adipocyte differentiation or adiponectin production according to an exemplary embodiment of the present disclosure may be a formulation for external application to skin. The term formulation for external application to skin is a general term including any formulation that can be applied externally to skin, and may include cosmetics and medications of various formulations.

In addition, the composition for promoting adipocyte differentiation or adiponectin production according to an exemplary embodiment of the present disclosure may be a food composition. The food composition may be a liquid or solid formulation, and may be a tablet, a capsule, a soft capsule, a pill, a granule, a drink, a diet bar, a chocolate, a caramel or confectionery, although not being particularly limited thereto. The food composition of the present disclosure may contain, in addition to the active ingredient, an excipient, a sugar, a fragrance, a colorant, an oil or fat, a protein, etc., as desired.

In an exemplary embodiment, the composition may further contain vitamin D. The composition, which further contains vitamin D, may supply the nutrient to hair follicles. Specifically, it may be contained in an amount of 0.0001-10 wt%, particularly 0.01-1 wt%, based on the total composition.

In other exemplary embodiments of the present disclosure, the present disclosure provides a method for promoting adipocyte differentiation or adiponectin production by administering an effective amount of a trimethoxyphenyl compound represented by Chemical Formula 1 to a subject in need thereof. This is not part of the invention as claimed.

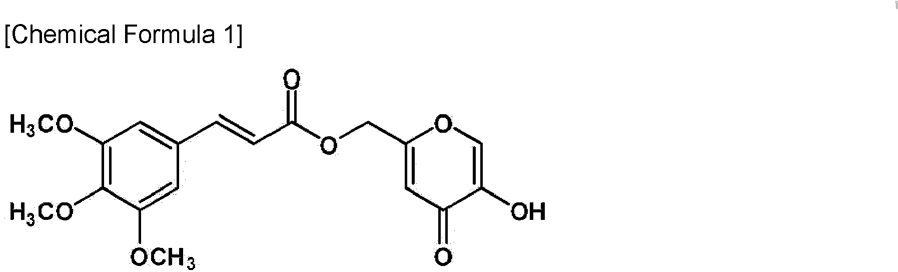

In other exemplary embodiments of the present disclosure, the present disclosure provides a use of a trimethoxyphenyl compound represented by Chemical Formula 1 for preparing a composition for promoting adipocyte differentiation or adiponectin production. This is not part of the invention as claimed.

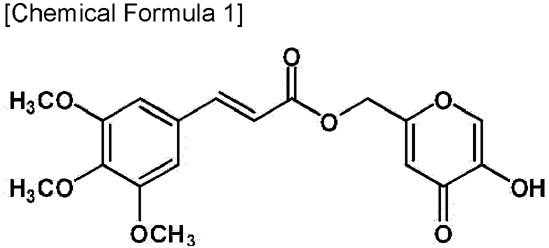

In other exemplary embodiments of the present disclosure, the present disclosure provides a trimethoxyphenyl compound represented by Chemical Formula 1 for promoting adipocyte differentiation or adiponectin production. This is not part of the invention as claimed.

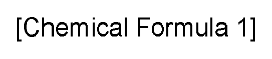

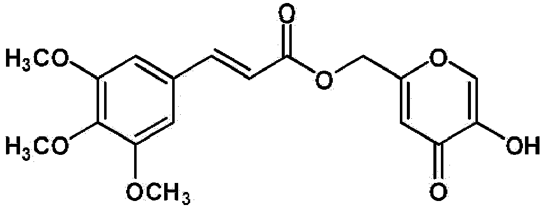

### [Mode for Invention]

Hereinafter, the present disclosure will be described in detail through examples.

### Preparation Example: Preparation of 3-(3,4,5-trimethoxyphenyl)-acrylic acid 5-hydroxy-4-oxo-4H-pyran-2-ylmethyl ester

3-(3,4,5-Trimethoxyphenyl)-acrylic acid 5-hydroxy-4-oxo-4H-pyran-2-ylmethyl ester as a trimethoxyphenyl compound of Chemical Formula 1 according to the present disclosure was prepared according to Scheme 2.

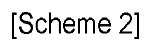

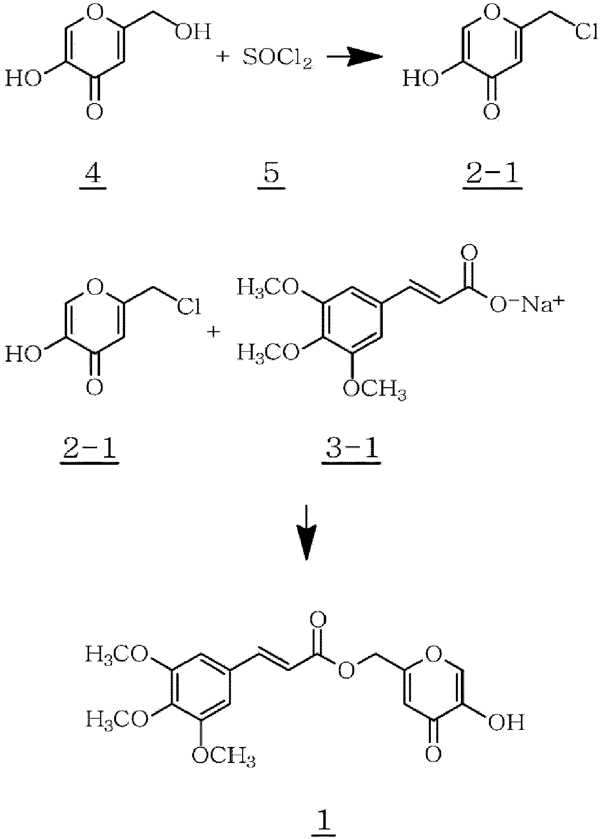

Details are as follows.

After dissolving 50 g of 5-hydroxy-2-(hydroxymethyl)-4H-pyran-4-one (0.35 mmol) in 250 mL of N,N-dimethylformamide and cooling in an ice-water bath at 10 °C, 50 g of thionyl chloride (0.42 mol) was added dropwise for 30 minutes. After stirring at room temperature for 2 hours, 2000 mL of ice water was added to the reaction solution. The produced solid was filtered and the (solid) filtrate was dissolved in 1000 mL of ethyl acetate. After drying by adding magnesium sulfate and activated carbon, followed by decoloration and filtering, the filtrate was concentrated and a crystal was obtained by adding hexane. After drying under reduced pressure, 39.5 g of 5-hydroxy-2-(chloromethyl)-4H-pyran-4-one of Chemical Formula 2-1 (70%) was obtained as a yellow solid.

Then, a compound of Chemical Formula 3-1 was prepared by dissolving 5 g of 3-(3,4,5-trimethoxyphenyl)-acrylic acid (0.026 mol) and 1.3 g of sodium hydroxide (0.031 mol) in 40 mL of methanol, distilling the methanol, and then dissolving the remaining residue in 70 mL of N,N-dimethylformamide.

After adding 4.2 g of the 5-hydroxy-2-(chloromethyl)-4H-pyran-4-one of Chemical Formula 2-1 (0.026 mol) to the compound of Chemical Formula 3-1, the mixture was heated in an oil bath at 110 °C for 2 hours under stirring. After distilling the solvent and dissolving the residue in 300 mL of ethyl acetate, the ethyl acetate solution was washed with 5% hydrochloric acid and distilled water, dried by adding magnesium sulfate and activated carbon, and then decolored. Then, 5.9 g of a reaction product (69% yield) was obtained as a pale-yellow solid by filtering the insoluble matter and evaporating the filtrate under reduced pressure.

The NMR analysis result of the pale-yellow solid is shown in FIG. 5.

### Examples

### Example 1: Proliferation of adipocytes and analysis of cytotoxicity

### Cell culturing and differentiation

Human subcutaneous fat cells (hereinafter, hSCFs) and a subcutaneous preadipocyte differentiation medium were purchased from ZenBio Inc. (NC, USA). The cells were cultured in a humidified 5% CO₂ incubator for 7 days.

To induce differentiation, the hSCFs were cultured further for 14 days in a Dulbecco's modified Eagle's medium (DMEM; Lonza, MD, USA) supplemented with 10% fetal bovine serum (FBS, PAA, Pasching, Austria), 10 µg/mL insulin (Sigma-Aldrich, MO, USA), 0.5 mM 3-isobutyl-1-methylxanthine (IBMX; Sigma-Aldrich, St. Louis, MO, USA), 1 µM dexamethasone (DEX, Sigma-Aldrich, St. Louis, MO, USA) and 1 µM troglitazone (Sigma-Aldrich, St. Louis, MO, USA) while replacing the medium every other day.

### Proliferation of cells and analysis of cytotoxicity

The viability of the hSCFs was measured using the EZ-Cytox cell viability assay kit (MTT assay, Daeil Lab Service, South Korea) according to the manufacturer's instructions. In brief, the hSCFs were cultured for 7 days in undifferentiated state, and then treated with each of 400 µM kojic acid (KA), 60 µM cinnamic acid (CA) and 30 µM glibenclamide (GC), as positive control groups, and the compound PAC-2 at various concentrations (µM) for 24 hours or 72 hours, respectively. Then, after adding the EZ-Cytox solution (10 µL) to each well, incubation was performed at 37 °C for 2 hours. Then, absorbance at 450 nm was measured using a spectrophotometer (Synergy H2, BioTek., VT, USA). All experiments were triplicated and the data are presented as the absorbance.

As a result, the compound according to the present disclosure showed comparable or better effect of proliferating hSCFs as compared to the positive control groups, kojic acid, cinnamic acid and glibenclamide, which suggests that adipocyte differentiation was promoted and this can lead to increased skin volume, elasticity, etc.

In addition, the cytotoxicity was comparable to the normal control group or not worse than the positive control groups, suggesting that the compound according to the present disclosure is not harmful to the human body.

### Example 2: Quantitative analysis of adipocyte differentiation

### Oil Red O (ORO) staining

The hSCFs cultured in Example 1 were differentiated for a total of 14 days by treating with a composition containing PAC-2 at various concentrations (µM) or with KA (400 µM), CA (60 µM) or GC (30 µM) as positive control groups. Then, after washing twice with cold PBS (phosphate-buffered saline), the cells were fixed in 3.7% formaldehyde (Sigma-Aldrich, St. Louis, MO, USA) for 1 hour. The fixed hSCFs were washed with 60% propylene glycol (Sigma-Aldrich, St. Louis, MO, USA) in PBS and were treated with Oil Red O (0.3 % Oil Red O in 60% propylene glycol; Sigma-Aldrich, St. Louis, MO, USA) for 30 minutes. The cells were washed with 85% propylene glycol thrice and then rinsed with tap water. Lipid droplets stained with the Oil Red O dye were visualized with an IX71 microscope (Olympus, Tokyo, Japan).

Referring to FIG. 2, when compared with the positive control groups, KA (400 µM), CA (60 µM) and GC (30 µM), the composition containing PAC-2 (indicated as PAC-16742 in the figure) showed a similar result at the concentration of 10 µM as the positive control groups, and showed remarkably increased number of stained cells at the concentrations of 25 µM and 50 µM. That is to say, the number of the stained cells was increased significantly as the concentration was increased. The result was also significant as compared to seletinoid G, which is known to promote adipocyte differentiation. Accordingly, it was confirmed that the composition containing the trimethoxyphenyl compound according to the present disclosure effectively promotes adipocyte differentiation.

### Example 3: Analysis of mRNA expression of adiponectin

### Real-time quantitative PCR (RT-qPCR)

Total RNA was extracted by treating the hSCFs cultured in Example 1 with a composition containing PAC-2 at various concentrations (µM) or KA (400 µM), CA (60 µM) and GC (30 µM) as positive control groups using a TRIzol reagent (Life Technologies, Carlsbad, CA, USA) according to the manufacturer's instructions. 1 µg of total RNA was utilized to synthesize complementary DNAs (cDNAs) using the RevertAid first strand cDNA synthesis kit (Thermo Scientific, Pittsburgh, PA, USA). About 1 µg of the cDNA sample and each TaqMan^{®} probe (Life Technologies, Carlsbad, CA, USA) were diluted in a reaction mixture of the Quantitect probe PCR kit (QIAGEN, Valencia, CA, USA) and PCR was conducted using the 7500 fast real-time PCR system (Life Technologies, Carlsbad, CA, USA). The TaqMan^{®} probe was glyceraldehyde-3-phosphate dehydrogenase (GAPDH; Part # 4352339E). All data were acquired from three independent experiments and are presented as fold changes relative to the GAPDH control group.

Referring to FIG. 3 showing mRNA expression level of adiponectin, when compared with the positive control groups, KA (400 µM), CA (60 µM) and GC (30 µM), the composition containing PAC-2 showed a similar result at the concentration of 10 µM in the mRNA expression level of adiponectin as the positive control groups, and showed 3 times or higher expression level at 25 µM and 5 times or higher expression level at 50 µM. That is to say, the expression level was increased significantly as the concentration was increased.

### Example 4: Quantitative analysis of adiponectin production

### Enzyme-linked immunosorbent assay (ELISA assay)

The hSCFs cultured in Example 1 were treated with KA (400 µM), CA (60 µM) or GC (30 µM) as positive control groups or a composition containing PAC-2 at various concentrations. The culture medium was collected and centrifuged at 13,000 rpm for 15 minutes to remove any debris. The secreted adiponectin was measured using an adiponectin ELISA kit (Enzo Life Sciences, Farmingdale, NY, USA) according to the manufacturer's instructions.

Referring to FIG. 4, when compared with the positive control groups, KA (400 µM), CA (60 µM) and GC (30 µM), the composition containing PAC-2 showed a similar result at the concentration of 10 µM in the amount of the secreted adiponectin as the positive control groups, and showed 3 times or higher amount at 25 µM and 7 times or higher amount at 50 µM. That is to say, the amount of the secreted adiponectin in the medium was increased significantly as the concentration was increased.

## Claims

1. A non-therapeutic use of a composition comprising a trimethoxyphenyl compound represented by Chemical Formula 1, a salt thereof, a hydrate thereof or a solvate thereof as an active ingredient: 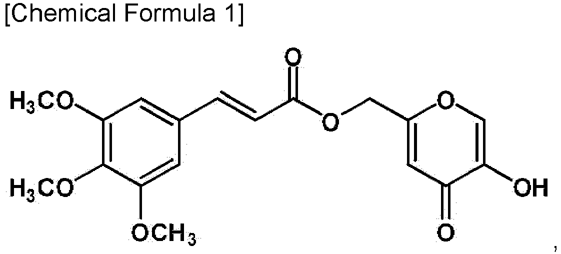 for enhancing volume or elasticity of skin by promoting adipocyte differentiation or adiponectin production.

2. The non-therapeutic use according to claim 1, wherein the concentration of the active ingredient is 1-100 µM based on the total volume of the composition.

3. The non-therapeutic use according to claims 1 or 2, wherein the composition is a cosmetic composition.

4. The non-therapeutic use according to any of claims 1-3, wherein the composition is a food composition.

5. The non-therapeutic use according to any of claims 1-4, wherein the composition further comprises vitamin D.

## Patentansprüche

1. Nicht therapeutische Verwendung einer Zusammensetzung, die eine durch die chemische Formel 1 dargestellte Trimethoxyphenyl-Verbindung, ein Salz davon, ein Hydrat davon oder ein Solvat davon als Wirkstoff umfasst: 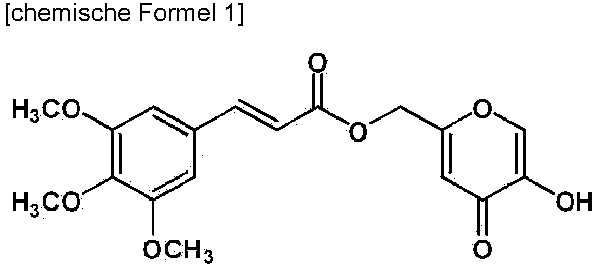 zur Verbesserung des Volumens oder der Elastizität der Haut, indem die Adipozytendifferenzierung oder die Adiponektinproduktion gefördert wird.

2. Nicht therapeutische Verwendung nach Anspruch 1, wobei die Konzentration des Wirkstoffs 1-100 µM beträgt, basierend auf dem Gesamtvolumen der Zusammensetzung.

3. Nicht therapeutische Verwendung nach Anspruch 1 oder 2, wobei die Zusammensetzung eine kosmetische Zusammensetzung ist.

4. Nicht therapeutische Verwendung nach einem der Ansprüche 1-3, wobei die Zusammensetzung eine Nahrungsmittelzusammensetzung ist.

5. Nicht therapeutische Verwendung nach einem der Ansprüche 1-4, wobei die Zusammensetzung ferner Vitamin D umfasst.

## Revendications

1. Utilisation non thérapeutique d'une composition comprenant un composé triméthoxyphényle représenté par la Formule Chimique 1, un sel de celui-ci, un hydrate de celui-ci ou un solvate de celui-ci en tant qu'ingrédient actif : 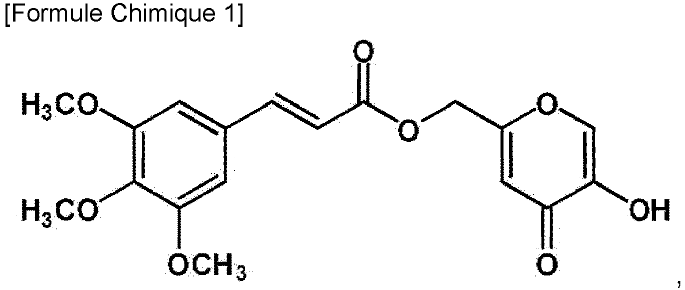 pour augmenter le volume ou l'élasticité de la peau en favorisant la différenciation des adipocytes ou la production d'adiponectine.

2. Utilisation non thérapeutique selon la revendication 1, dans laquelle la concentration de l'ingrédient actif est de 1 à 100 µM par rapport au volume total de la composition.

3. Utilisation non thérapeutique selon les revendications 1 ou 2, dans laquelle la composition est une composition cosmétique.

4. Utilisation non thérapeutique selon l'une des revendications 1 à 3, dans laquelle la composition est une composition alimentaire.

5. Utilisation non thérapeutique selon l'une des revendications 1 à 4, dans laquelle la composition comprend en outre de la vitamine D.
